# EUROPEAN PATENT SPECIFICATION

(11) **EP 0 891 770 B1**
(45) Date of publication and mention of the grant of the patent: **28.08.2002**
(21) Application number: 98109072.3
(22) Date of filing: 19.05.1998
(51) Int. Cl.: A61K 9/70, A61K 39/00, A61K 39/205, A61K 39/245

(54) **Killer T cell activator and use thereof**
Killer-T-Zell Aktivator und dessen Verwendung
Activateur des cellules du type T tueuses et son utilisation

(30) Priority: 20.05.1997 JP 13003197
(43) Date of publication of application: 20.01.1999
(73) Proprietor: Nitto Denko Corporation, Ibaraki-shi, Osaka 567-0041 (JP)
(72) Inventor: Takigawa, Masahiro, Kyoto-shi, Kyoto 603-8047 (JP); Seo, Naohiro, Hamamatsu-shi, Shizuoka 431-3124 (JP)
(74) Representative: von Kreisler, Alek, Dipl.-Chem.

(56) References cited:
- DE-A- 19 525 607
- E. PROKSCH: "Bedeutung der Permeabilitätsbarriere der Haut für das irritativ-toxische und das allergische Kontaktekzem (Significance of epidermal permeability barrier in irritant and allergic contact dermatitis)." ALLERGOLOGIE, vol. 17, no. 8, August 1994, pages 346-349, XP002076342 Munich, Germany
- DATABASE WPI Week 8244 Derwent Publications Ltd., London, GB; AN 82-94140E XP002076346 & JP 57 156414 A (SEKISUI CHEMICAL INDUSTRIES CO. LTD.), 27 September 1982
- B. NICKOLOFF ET AL.: "Perturbation of epidermal barrier function correlates with initiation of cytokine cascade in human skin." JOURNAL OF THE AMERICAN ACADEMY OF DERMATOLOGY, vol. 30, no. 4, April 1994, pages 535-546, XP002076343 St. Louis, MO, USA
- M. DE BRUIJN ET AL.: "Processing of exogenous protein antigen by murine dendritic cells for presentation to cytotoxic T lymphocytes." ADVANCES IN EXPERIMENTAL MEDICINE AND BIOLOGY, vol. 417, 1997, pages 213-220, XP002076344 New York, NY, USA
- PATENT ABSTRACTS OF JAPAN vol. 017, no. 482 (C-1105), 2 September 1993 & JP 05 123326 A (NEC CORP), 21 May 1993
- T. NISHIJIMA ET AL.: "Altered permeability and disordered cutaneous immunoregulatory function in mice with acute barrier disruption." THE JOURNAL OF INVESTIGATIVE DERMATOLOGY, vol. 109, no. 2, August 1997, pages 175-182, XP002076345 Baltimore, MD, USA

## Description

The present invention relates to a pharmaceutical preparation in the form of a plaster comprising a protective antigen, which is suitable for the activation of killer T cells having specificity for said protective antigen, and for the prophylaxis and treatment of cancer and diseases caused by microbial infection.

The present invention further relates to use of a protective antigen for the production of a protective antigen-specific killer T cell activator.

### BACKGROUND OF THE INVENTION

As of date, there is no medication or treatment method for intractable diseases causing serious conditions of patients, such as cancer and viral infectious diseases (e.g., acquired immune deficiency syndrome (AIDS), hepatitis B and hepatitis C ), which selectively attacks and eradicates only the pathogenic cells, and an effective treatment method is needed, which is free of side effects and adverse influence on the living body.

Among potential methods for selectively eradicating malignant cells and cells infected with virus is a method based on activation of the immune system that a living body innately possesses, and one of such known methods for inducing activation of mammalian immune system is vaccination. Vaccines are divided into live vaccines containing live attenuated microorganisms and inactivated vaccines containing inactivated microorganisms or infection-protective antigens thereof. A component vaccine, which is an inactivated vaccine, is prepared using, as an antigen, a microbial antigenic protein, a surface protein of malignant cell or a peptide fragment thereof, so that it can be used as an effective means for activating an immune system with safety. However, existing vaccination only activates humoral immunity and increases production of antigen-specific antibodies. This method is effective for removing viral particles released from cells and the like, but ineffective for eliminating malignant cells and infected cells causing viral infections.

Of the immune systems in the living body, CD 8 molecule-positive killer T cells (hereinafter also referred to as CTL) play an important role in the selective eradication of virus-infected cells and malignant cells. To be specific, virus-infected cells process viral proteins, and malignant cells process mutated proteins, respectively, within the cytoplasm into peptide fragments, and express the fragments on the cell surface on a "vessel" called major histocompatibility complex (hereinafter abbreviated as MHC) class I molecule, thereby notifying the immune system in a living body of the abnormal nature of the cells. CTL recognize said viral peptide or mutated peptide (carcinoma antigen peptide)/MHC class I molecule complex and eradicate the cells that expressed the complex.

In recent years, vaccinations aiming at activation and amplification of CTL, which specifically recognize and eradicate virus-infected cells and malignant cells, have been actively studied. Among others, dendritic cells known to have antigen presenting capability to helper T cells (Th) have been elucidated to have high antigen presenting capability to CTL as well via MHC class I molecule, and the development of vaccination using the dendritic cells has been drawing much attention [J. I. Mayordomo et al. *Nature Med*., *1* (12), 1279-1302 (1995)]. This method, nevertheless, requires complicated manipulations, such as isolation of progentors of dendritic cells and a long term culture, since it involves isolation of progentors of dendritic cells present in peripheral blood in a slight amount, a long term *in vitro* culture thereof for proliferation, preparation of resultant activated and differentiated dendritic cells with viral peptide or carcinoma antigen peptide to induce dendritic cells, said dendritic cells presenting antigenic peptide on the MHC class I molecule on the cell surface, and transfer thereof into the living body. Such complicated manipulation makes the possibility of its clinical application scarce.

Moreover, E. Proksch, Allergologie, 17(8), 346-349 (1994) reports on the activation of the immune system and an increase in the number of Langerhans cells due to increased penetration of antigens into the epidermis under conditions of acute permeability barrier disruption. This is discussed in the context of i.a. contact dermatitis.

It is therefore an object of the present invention to provide a pharmaceutical preparation which efficiently activates CTL that selectively eradicate cells infected with virus or malignant cells, for the establishment of an effective method for the prophylaxis and treatment of cancer and diseases caused by viral infection.

### SUMMARY OF THE INVENTION

In accord with the present invention, it has now been found that Langerhans cells, which are dendritic cells constantly present in a great number in cutaneous epidermis, can be activated by merely disrupting a stratum comeum of the epidermis mechanically or chemically, the stratum being a primary barrier in the living body against exogenous stimulus, thus resulting in pronounced enhancement of antigen presenting capability to CTL, and that the activated epidermal Langerhans cells show increased expression of MHC class I molecule responsible for antigen presentation to CTL, as well as increased expression of ICAM-1, B7-2, and CD 40 required for an ensured adhesion to CTL, followed by transfer of the Langerhans cells into the lymph node where a number of CTL precursor cells are present. It has been also found that when an antigenic peptide is percutaneously inoculated by coating a viral peptide or carcinoma antigen peptide to cutaneous epidermis having disrupted stratum corneum barrier, or by applying a pressure sensitive adhesive tape containing said antigenic peptide in an adhesive layer in a releasable manner, within 24 hours after barrier disruption, epidermal Langerhans cells move into the lymph node while presenting said antigen on MHC class I molecule on the cell surface showing an increased expression of the MHC class I molecule. In the lymph node, the cells adhere to CTL precursor cells to activate and amplify CTL precursor cells. Consequently, the precursor cells can be efficiently differentiated into said antigen-specific mature CTL. In addition, it has been confirmed that the lymphocytes, taken from mammals percutaneously inoculated with antigenic peptide by the above-mentioned method, selectively lyse only cells pulsed with said antigen.

That is, the present invention provides the following.
(1) A protective antigen-specific CTI activator in the form of a plaster, which comprises the protective antigen and a pharmaceutically acceptable carrier, the activator being suitable for an external application to an epidermal Langerhans cell surface activated by mechanically or chemically disrupting the stratum corneum of the epidermis.
(2) The activator of (1) above, wherein said plaster comprises a support and an adhesive layer comprising the protective antigen in a releasable manner, said adhesive layer being laminated on one side of the support.
(3) The activator of (2) above, wherein the surface of the adhesive layer is divided into two areas and either area comprises the protective antigen.
(4) The activator of (1) above, further comprising a support and adhesive layers laminated on both sides of the support, wherein the adhesive layer on one side comprises the protective antigen in a releasable manner.
(5) The activator of (1) above, wherein the protective antigen is selected from a pathogenic microorganism, a peptide to be an infection-protective antigen thereof and a malignant cell-specific mutated peptide.
(6) The activator of (5) above, which is an agent for the prophylaxis and treatment of cancer or a disease caused by microbial infection.
(7) Use of a protective antigen for the production of a protective antigen-specific CTL activator which is suitable to be externally applied to the surface of epidermal Langerhans cells activated by mechanically or chemically disrupting a stratum corneum of the epidermis.
(8) The use of (7) above, wherein the activator is in the form of a plaster.
(9) The use of (8) above, wherein the plaster comprises a support and an adhesive layer comprising the protective antigen in a releasable manner, said adhesive layer being laminated on one side of the support.
(10) The use of (9) above, wherein the surface of the pressure sensitive adhesive tape is devided into two areas and the protective antigen is contained in one of the areas.
(11) The use of (8) above, further comprising the use of a support and adhesive layers laminated on both sides of the support, said adhesive layer on one side comprising the protective antigen in a releasable manner, wherein the activator is in the form of a pressure sensitive adhesive tape.
(12) The use of (7) above, wherein the protective antigen is at least one member selected from a pathogenic microorganism, a peptide to be an infection-protective antigen thereof and a malignant cell-specific mutated peptide.
(13) The use of (7) above, wherein the activator is suitable for the prophylaxis and treatment of cancer or a disease caused by microbial infection.

### BRIEF DESCRIPTION OF THE DRAWINGS

Fig. 1 shows time-course changes in the number of mouse earlobe Langerhans cells after disrupting the stratum corneum by tape stripping.
Fig. 2 shows changes in the expression of accessory molecule in mouse earlobe Langerhans cells after disrupting the stratum comeum by tape stripping.
Fig. 3 shows changes in the expression of H-2K^{b} molecule on the surface of mouse earlobe Langerhans cells after disrupting the stratum corneum by tape stripping.
Fig. 4 shows expression of H-2K^{b} molecule in L^{kb} cell.
Fig. 5 shows cytotoxic activity of cervical lymphocyte-derived effector cells obtained from mice, to which three kinds of antigenic peptides were applied separately at 12 hours after disrupting an earlobe stratum corneum by tape stripping.
Fig. 6 shows cytotoxic activity of cervical lymphocyte-derived effector cells obtained from mice, to which three kinds of antigenic peptides were applied separately at 12, 24 and 48 hours after disrupting an earlobe stratum corneum by tape stripping.

### DETAILED DESCRIPTION OF THE INVENTION

The inventive method for the activation of protective antigen-specific CTL is based on the phenomenon that mechanical or chemical disrupting of stratum corneum of the epidermis results in an increased expression of MHC class I molecule on the surface of epidermal Langerhans cells, which is accompanied by transfer of the activated epidermal Langerhans cells to lymph node. Once a protective antigen is inoculated percutaneously to said epidermis during the transfer of said epidermal Langerhans cells to the lymph node after an increased expression of MHC class I molecule, an auto peptide in the MHC class I molecule is substituted by a peptide of the protective antigen.

In the present invention, by the protective antigen is meant not only microbial proteins which become infection protective antigens of pathogenic microorganisms (e.g., bacteria, fungi, and virus), mutated proteins specifically expressed in malignant cells, and immunogenic peptide fragments thereof, but also live or dead attenuated microorganisms conventionally used as live vaccines or inactivated vaccines. The microbial proteins are exemplified by HIV-derived protein, surface antigens of hepatitis B and C viruses, and surface antigen of influenza virus. The malignant cell-specific mutated protein is exemplified by malignant transformation cell differentiation antigen protein. Said protective antigen may consist of a single protective antigen or plural protective antigens to be mixed for use.

The activated epidermal Langerhans cells move into lymph node while presenting antigen by an MHC class I molecule/protective antigen peptide complex, and strictly bind to CTL precursor cell having MHC-restricted T cell receptor which specifically recognizes said protective antigen in the lymph node. The bond between the both cells may be an antigen-receptor bond, MHC class I molecule-CD 8 bond, ICAM-1-LFA-1 bond or LFA-3-CD 2 bond. The bond activates CTL precursor cells to cause amplification and differentiation to antigen-specific mature CTL. The maturated and amplified antigen-specific CTL patrol the entire body on lymph circulation. When they recognize the target cell (e.g., virus-infected cell and malignant cell) which shows said antigen, they bind thereto and release cytotoxic substance, such as perforin and granzyme, to kill the target cell.

While the method for disrupting the stratum corneum is not particularly limited, it is exemplified by a method comprising removing sebum, which forms intercellular space of the stratum corneum, by the action of an organic solvent such as acetone, and a method comprising mechanically disrupting the stratum corneum. A preferable example of the latter is a method wherein a pressure sensitive adhesive tape is adhered to the surface of a stratum corneum of the epidermis and stripped therefrom (hereinafter the method is referred to as pressure-sensitive adhesive tape stripping) in one or more cycles. [Bunya Shirai et al., Nitto Technical Report, 31 (2), 76(1993)].

The pressure sensitive adhesive tape to be used for stripping preferably has a peel adhesion of not less than 80 g/12 mm width relative to a Bakelite board. Considering no difference in effects caused by peeling of keratin more than necessary, the peel adhesion is preferably not more than 1600 g/12 mm width. The adhesive may be any such as an acrylic polymer adhesive and a rubber polymer adhesive

The percutaneous inoculation of the protective antigen is performed by administering the inventive antigen-specific CTL activator to the epidermis surface, from which a pressure sensitive adhesive tape was stripped, simultaneously with the disruption of stratum corneum or within 24 hours, preferably within 12 hours, after the disruption. The inventive antigen-specific CTL activator is subject to no particular limitation in the dosage form as long as it is an external agent, and typically contains a protective antigen and a suitable base for external agent. Specific examples include an ointment prepared by admixing a protective antigen with a suitable base for an ointment and a pressure sensitive adhesive tape designed to permit sustained release of the protective antigen, which contains an adhesive layer as a base and the protective antigen inoculated to or embedded in said adhesive layer (hereinafter to be referred to as a pressure sensitive adhesive tape for antigen-specific CTL activation).

In the case of an ointment, the base for an ointment may be, for example, fat and oil type base, such as petrolatum, paraffin, plastibase, silicone, vegetable oil, and wax ; emulsion base such as hydrophilic ointment, hydrophilic petrolatum, purified lanolin and the like; water soluble base such as macrogols. Where necessary, a preservative such as p-hydroxybenzoate may be added.

The protective antigen can be percutaneously inoculated by applying said ointment to the skin surface where the stratum corneum has been disrupted. The protective antigen content of the ointment is preferably 0.1 µmol/cm² -1 mmol/cm² of one kind of antigen when the ointment is applied in a typical amount.

A particularly preferable embodiment of the percutaneous inoculation of the protective antigen is that using the inventive pressure sensitive adhesive tape for activation of antigen-specific CTL, which is to be applied to the skin surface where the stratum corneum has been removed. The inventive pressure sensitive adhesive tape for antigen-specific CTL activation is designed to permit appropriate release of the protective antigen and to stabilize said protective antigen which has been inoculated to the surface of the adhesive layer or embedded therein, to the degree that the function as the original protective antigen is retained. The inventive pressure sensitive adhesive tape for antigen-specific CTL activation preferably has an adhesive power which is great enough to sufficiently adhere the tape to the skin surface. The adhesive to be used is essentially required not to lower or vary the antigenicity of the protective antigen. As long as such condition is met, the adhesive may be an acrylic polymer adhesive or a rubber polymer adhesive. Particularly preferable adhesive is that made from a hydrophilic polymer.

Examples of the hydrophilic polymer include water soluble natural polymers such as gum arabic and carboxymethylcellulose, polyvinylpyrrolidone, polyvinyl alcohol, polymethoxyethyl acrylate, polyacrylic acid and the like, which are obtained by polymerization of water soluble polymer such as vinyl pyrrolidone, vinyl alcohol, 2-hydroxyethyl acrylate, 2-methoxyethyl acrylate and acrylic acid, and copolymers of two or more of these water soluble monomers. It may be an adhesive polymer obtained by polymerization of hydrophobic polymer such as butyl acrylate and 2-ethoxyhexyl acrylate to the extent that the hydrophilicity required in the present invention is not impaired.

More preferable hydrophilic polymer is exemplified by an acrylic copolymer comprising alkoxyalkyl acrylate and N-vinyl lactam. Alkoxyalkyl acrylate, which is a monomer component of the acrylic copolymer, is preferably an acrylate comprising an alkoxy having 1 to 4 carbon atoms and alkyl or alkylene glycol having 2 to 4 carbon atoms. Specific examples include alkoxyalkyl acrylate such as 2-methoxyethyl acrylate, 2-ethoxyethyl acrylate, 2-butoxyethyl acrylate, 3-methoxypropyl acrylate, 3-ethoxypropyl acrylate, and alkoxyalkylene glycol acrylate such as methoxytriethylene glycol acrylate, methoxydipropylene glycol acrylate and the like, with preference given to 2-methoxyethyl acrylate and 2-ethoxyethyl acrylate, in view of high hydrophilicity.

The other monomer component, N-vinyl lactam, may be N-vinyl lactam which is a 5 to 7-membered ring. Examples thereof include N-vinyl-2-pyrrolidone, N-vinyl-2-piperidone, N-vinyl-2-caprolactam with preference given to N-vinyl pyrrolidone in view of safety and general applicability.

While the alkoxyalkyl acrylate content is not particularly limited, it is preferably 60-80 wt%, more preferably 65-75 wt%, of the entire acrylic copolymer. While the N-vinyl lactam content is not particularly limited, it is preferably 20-40 wt%, more preferably 25-35 wt%, of the entire acrylic copolymer.

The hydrophilic polymer can be prepared by a method known *per se*. For example, acrylic copolymer can be produced by any copolymerization method such as solution polymerization, emulsion polymerization, suspension polymerization bulk polymerization, and optical polymerization.

The adhesive layer may further contain a hydrophilic or water soluble low molecular substance to achieve a suitable degree of adhesion power. The hydrophilic or water soluble low molecular substance may be a liquid compound having a high boiling point of 100-400°C. Examples thereof include polyhydric alcohol, and sugar alcohol with preference given to a reducing sugar free of browning reaction (Maillard reaction) with a protein. The polyhydric alcohol is exemplified by ethylene glycol, diethylene glycol, triethylene glycol, liquid polyethylene glycol, propylene glycol, dipropylene glycol, 1,1,1-trihydroxpropane, glycerol and the like, and the reducing sugar is exemplified by sorbitol, sorbitan, erithyritol, xylitol, and trehalose.

Alternatively, adducts with ether of glycerol and ethylene glycol, propylene glycol and the like, such as polyoxyethyleneglycerol ether, adducts with ether of sorbitol or sorbitan and ethylene glycol, propylene glycol and the like, such as polyoxypropylenesorbitol ether and polyoxyethylenesorbitan ether may be used. These hydrophilic or water soluble low molecular substances can be added in an amount within the range of from 5 wt% to 90 wt% of the adhesive constituting the adhesive layer.

In the above-mentioned hydrophilic adhesive layer, the entire adhesive layer may be crosslinked, wherein 5-75% of the components constituting said adhesive layer may be a gel-like insoluble matter which does not dissolve in water. In this case, the adhesive layer may be crosslinked by the addition of a crosslinking agent The crosslinking agent may be a polyhydric epoxy compound such as ethylene glycol diglycidyl ether, triglycidyl isocyanulate and the like, or an isocyanate compound such as CORONATE L and CORONATE HL (Japan Polyurethane Corp.). These crosslinking agents can be added in an amount of 0.01-5 parts by weight per 100 parts by weight of the adhesive constituting the adhesive layer.

A different crosslinking method of the adhesive layer may be insolubilization by exposure to electron beam or γ rays. The radiation in the case of, for example, acrylic copolymer may be not less than 1 Kgy, particularly preferably not less than 25 Kgy and not more than 50 Kgy.

The adhesive layer is subject to no particular limitation as long as it has sufficient adhesive power to stay on the skin surface of a living body. For example, it has a peel adhesion of not less than 30 g/12 mm width, preferably not less than 80 g/12 mm width, relative to a Bakelite board. When it adheres unnecessarily strong to the skin of a living body, the skin tissue may be disrupted. Thus, it is preferably designed to have a peel adhesion of not more than 600 g/12 mm width. The thickness of the adhesive layer is such as to afford necessary peel adhesion and to be free of cohesive failure of adhesive layer upon peeling, which is typically 10-100 µm, preferably 20-80 µm.

The inventive pressure sensitive adhesive tape for antigen-specific CTL activation can be in any dosage form depending on+ use, as long as it contains a protective antigen in at least part of the adhesive layer in a releasable manner. When said pressure sensitive adhesive tape is solely used for percutaneous inoculation of a protective antigen, it may comprise an adhesive layer laminated on one side of a support, wherein the adhesive layer contains the protective antigen preferably uniformly in the entirety thereof. When the pressure sensitive adhesive tape is used for both pressure sensitive adhesive tape stripping aiming at disrupting the stratum corneum and percutaneous inoculation of a protective antigen, the tape may comprise an adhesive layer laminated on one side of a support, wherein the adhesive layer is divided into two areas, one of which containing the protective antigen, or the tape may comprise adhesive layers laminated on both sides of a support, wherein only one of the adhesive layers contains the protective antigen. In either dosage form, an adhesive layer area without a protective antigen is used for pressure sensitive adhesive tape stripping and the adhesive layer area containing the protective antigen is used for percutaneous inoculation of the protective antigen. The both adhesive layer areas may be the same or different as long as they satisfy the above-mentioned requirements of pressure sensitive adhesive tape stripping and percutaneous inoculation of protective antigen.

The support to be used in the present invention is a flexible sheet and is not limited to a specific material as long as it is strong enough to stand damage during handling. Examples thereof include plastic films made from polyethylene, polypropylene, polyester, polyamide, polycarbonate, polysulfone, polyvinyl chloride, polyether, polyurethane, ethylene-vinyl acetate copolymer, acetate cellulose, and nitrocellulose.

The inventive pressure sensitive adhesive tape for antigen-specific CTL activation is prepared by, for example, adding an aqueous dispersion or a water/alcohol mixed dispersion adjusted to contain a protective antigen in a desired concentration to an aqueous solution of the above-mentioned adhesive hydrophilic polymer and a hydrophilic low molecular substance, or a water/alcohol mixture, followed by thorough mixing and dispersing. Then, the dispersion is applied on a flexible support sheet in a certain thickness and dried at a suitable temperature in the range of from 10°C to 200°C. The drying temperature is preferably as low as possible, so that degeneration of the contents of the viscous dispersion can be prevented, which is typically 30-100°C. The adhesive layer of the pressure sensitive adhesive tape produced by this method now contains a protective antigen uniformly at a desired concentration. In addition, since the entirety or part of the protective antigen is embedded in the adhesive layer thus formed, the protective antigen is caused to be released in an appropriately sustained manner. A different production method of the inventive pressure sensitive adhesive tape for antigen-specific CTL activation includes applying the above-mentioned aqueous dispersion or water/alcohol mixed dispersion containing an adhesive hydrophilic polymer and a hydrophilic low molecular substance onto a flexible support sheet in a certain thickness, and drying same at a suitable temperature of 10-200°C, and where necessary, crosslinking to give a pressure sensitive adhesive tape without a protective antigen, which is followed by application of an aqueous dispersion or a water/alcohol mixed dispersion adjusted to contain a necessary amount of a protective antigen onto the surface of the adhesive layer of said pressure sensitive adhesive tape in a necessary inoculation amount of the protective antigen and evaporation of water or alcohol. A pressure sensitive adhesive tape having an adhesive layer area containing the protective antigen and an area that does not can be prepared by suitably combining the above-mentioned two production methods.

The inventive pressure sensitive adhesive tape for antigen-specific CTL activation is preferably designed to contain a protective antigen in an amount corresponding to 0.1 µmol/cm²-1 mmol/cm² per antigen.

The inventive antigen-specific CTL activator sensitizes and amplifies the antigen-specific CTL by the aforesaid stratum corneum disruption and the subsequent percutaneous inoculation of the protective antigen. While circulating through lymphoid, the resulting antigen-specific CTL specifically recognize and bind to cells (e.g., virus-infected cell and malignant cell) presenting said protective antigen and eradicate such cells. Thus, the inventive antigen-specific CTL activator can be used for the prophylaxis and treatment of cancer and diseases caused by microbial infection with, for example, virus.

The inventive antigen-specific CTL activator is generally applicable to animals having a T cell dependent cellular immune system. It is most effectively used for mammals such as human, monkey, cow, horse, dog, cat, sheep, goat, rabbit, mouse, rat, hamster, and guinea pig.

Inasmuch as the inventive antigen-specific CTL activator specifically recognizes and binds to cells having a protective antigen and efficiently sensitizes and amplifies CTL capable of eradication of such cells, by a simple method involving disruption of stratum corneum of the epidermis and subsequent percutaneous inoculation of the protective antigen by antigen-specific CTL activator, it is extremely useful for the prophylaxis and treatment of intractable cancer and diseases caused by viral infection. The present invention also provides a drastic method for the prophylaxis and treatment of cancer and diseases caused by viral infection in mammals inclusive of human.

The present invention is described in more detail by the following illustrative Examples.

### Example 1

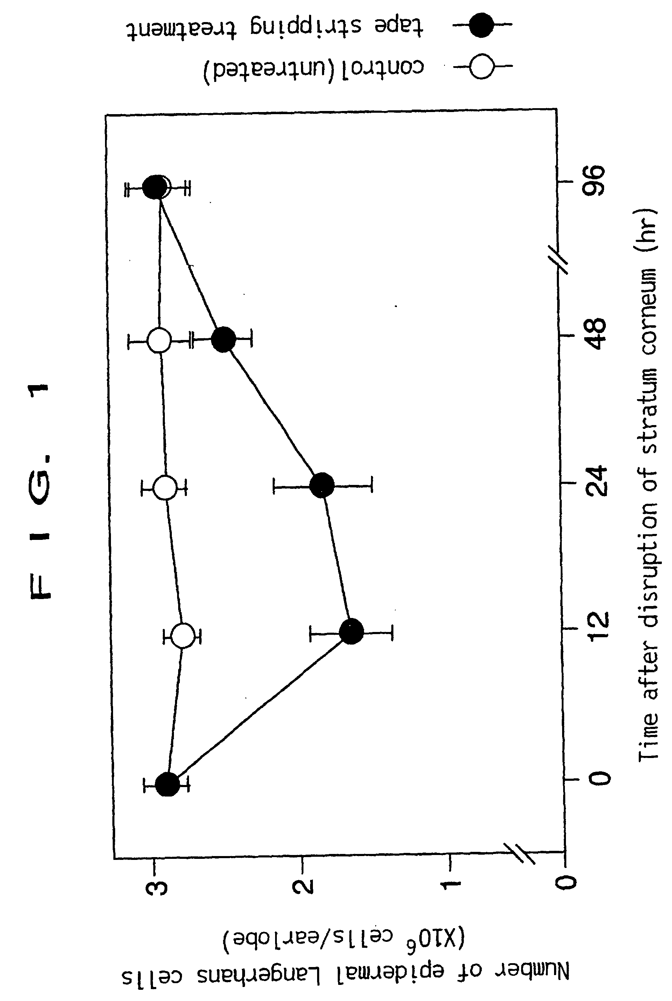

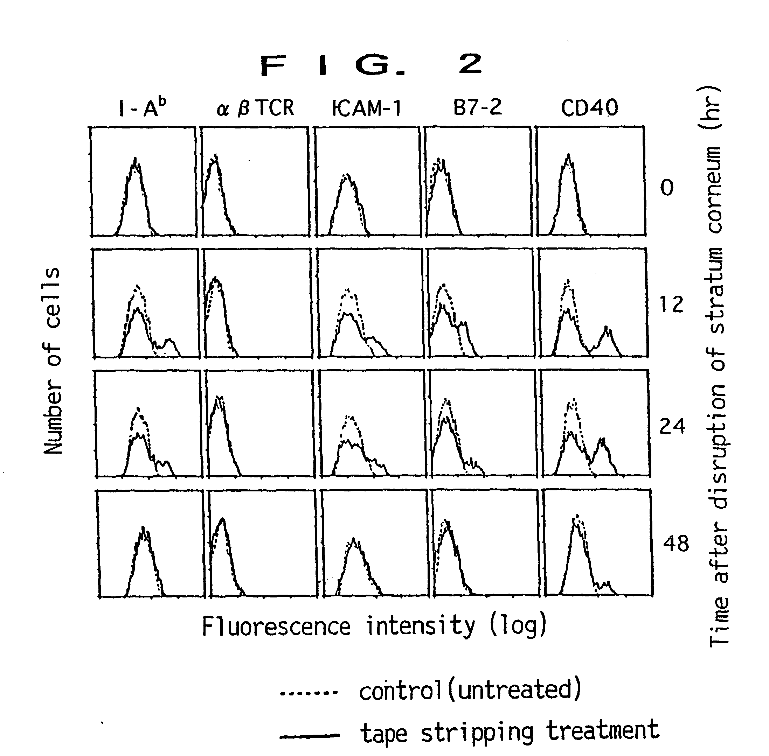

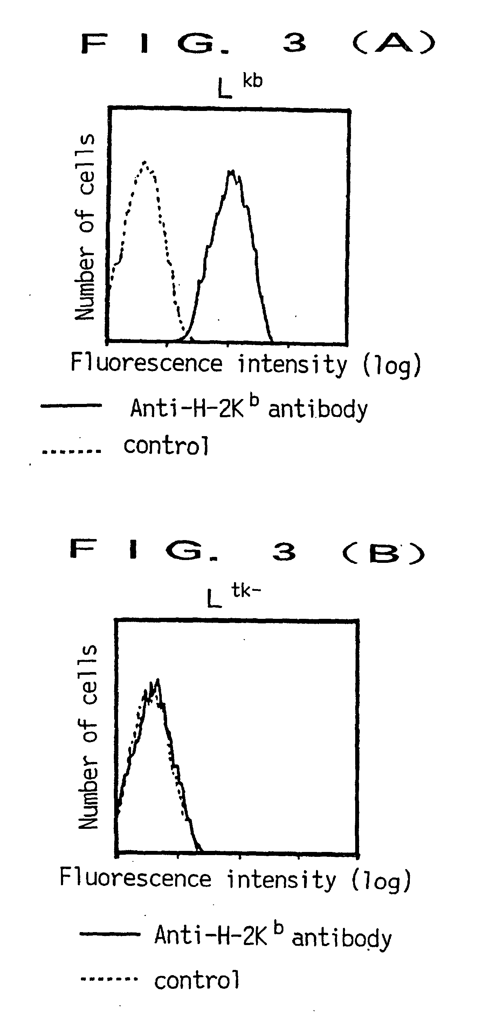

(1) Changes in epidermal Langerhans cell counts after removing the barrier of stratum corneum of the epidermis by pressure sensitive adhesive tape stripping.
   The keratin of one side of earlobe of a C57BL/6 (B6) mouse was disrupted by 10 repeats of stripping with a commercially available cellophane tape, and epidermal cell suspensions were obtained by trypsinizing the earlobe cut into a certain area at 12, 24 and 48 hours later. The suspensions were stained with I-A^{b} antigen-specific antibody, which is one of the MHC class II antigens, to count I-A^{b} antigen strong positive Langerhans cells by flow cytometry. As a control, the other earlobe of the mouse free of tape stripping was used. The results are shown in Fig. 1.
   The mouse earlobe free of pressure sensitive adhesive tape stripping showed normal Langerhans cell counts, whereas the cell count of the earlobe which underwent stripping decreased to about half in 12 hours after the tape stripping, thereby showing transit thereof to other tissues from epidermis. The transit of the Langerhans cells reached maximum at about 12-24 hours after tape stripping, and epidermal Langerhans cell count gradually restored the normal level.
(2) Expression of activated accessory molecule of epidermal Langerhans cell after disrupting stratum corneum.
   One earlobe of a B6 mouse was disrupted by 10 repeats of stripping with the same adhesive tape as used in Example 1-(1), and epidermal cell suspensions were obtained by trypsinizing the epidermis cut into a certain area at 0, 12, 24 and 48 hours later. The suspensions were stained with antibodies specific to I-A^{b}, CD54 (ICAM-1), CD86 (B7-2) and α βTCR, and analyzed by flow cytometry. As a control, the other earlobe of the same mouse, which was free of tape stripping, was used. The results are shown in Fig. 2.
   The earlobe Langerhans cells which underwent pressure sensitive adhesive tape stripping were activated 12 and 24 hours later, and showed an increased expression of ICAM-1, B7-2 and CD40 molecules responsible for binding with CTL. The staining using anti-α βTCR antibody as a control antibody did not result in enhanced expression after barrier disruption. The expression of ICAM-1, B7-2 and CD40 molecules reached its peak at about 12-24 hours after barrier disruption, and returned to the level before activation at 48 hours later. The mouse earlobe free of pressure sensitive adhesive tape stripping showed no variation in the expression level of ICAM-1, B7-2 and CD40 molecules of Langerhans cell.
(3) Expression of MHC class I molecule on the epidermal Langerhans cell surface after stratum corneum barrier disruption.
   In the same manner as in Example 1-(2), the expression of H-2K^{b} molecule, which is one of the MHC class I molecules, was examined. The results are shown in Fig. 3. The epidermal Langerhans cell of the mouse earlobe which underwent pressure sensitive adhesive tape stripping apparently showed an increased expression of H-2K^{b} molecule in about 12-24 hours after barrier disruption, which molecule being responsible for presentation of antigen to CTL (Fig. 3(A)). On the other hand, the Langerhans cell of earlobe free of stratum corneum disruption showed no reinforcement of expression of this molecule (Fig. 3(B)).

### Reference Example 1: Synthesis of antigenic peptide

In accord with a known literature, herpes simplex virus (HSV), vesicular stomatitis virus (VSV)-derived antigenic peptide, and ovalbumin (OVA)-derived peptide, all capable of being presented on H-2K^{b} molecule, which is one of the MHC class I molecules, and activation of CTL in B6 mouse, were synthesized.

### HSV glycoprotein B 498-505

[reference: Bonneau et al., Virology, 195:62-70(1993)] sequence: Ser Ser Ile Glu Phe Ala Arg Leu (SEQ ID NO: 1)

### VSV NP 53-59

[reference: Van Bleek and Nathenson, Nature, 348: 213-215 (1990)] sequence: Arg Gly Tyr Val Tyr Gln Gly Leu (SEQ ID NO: 2)

### Ovalbumin 257-264

[reference: Falo et al., Nature Med., 1: 649-653 (1995)] sequence: Ser Ile Ile Asn Phe Glu Lys Leu (SEQ ID NO: 3)

### Reference Example 2: Preparation of transformed L cell which expresses H-2K^{b} molecule

A cell line was prepared, wherein H-2K^{b} molecule had been forcibly expressed in L cell inherently having no H-2K^{b} molecule, for use in *in vitro* confirmation of whether or not CTL, which are activated in a mouse when antigenic peptide is applied to the tape-stripped skin, is specific to H-2K^{b} molecule which presents antigenic peptide.

### (i) Preparation of cell line transformed with H-2K^{b} molecule gene

A plasmid DNA, containing an H-2K^{b} gene inserted in the downstream of a known promoter functional in L cell and having a tymidine kinase gene, was prepared. The plasmid DNA was introduced into L^{tk-} cell by a calcium chloride method. The cells having the gene were screened using tymidine kinase activity as an index to obtain a cell line having an L^{kb} gene.

### (ii) Verified expression of H-2K^{b} molecule in L^{kb} cell

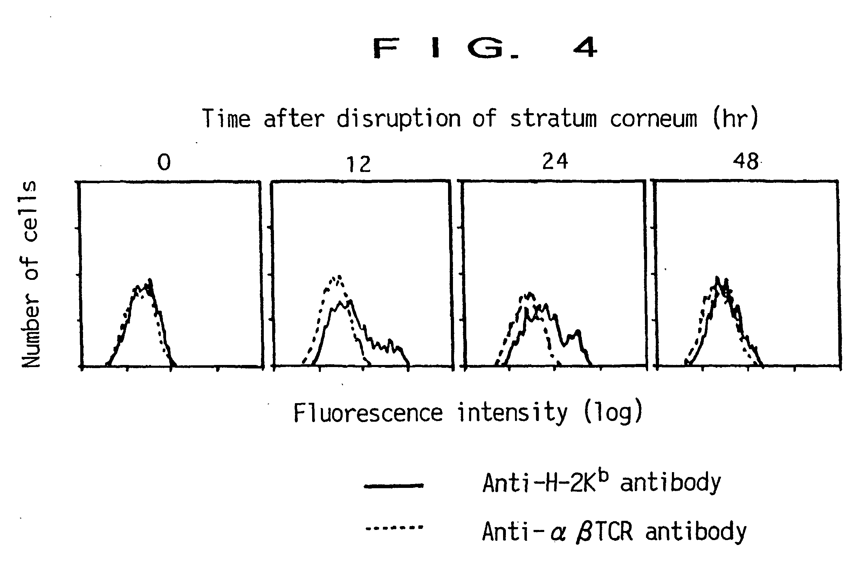

The prepared L^{kb} cell was stained with H-2K^{b}-specific antibody and analyzed by flow cytometry. As a control of the cell, L^{tk-} cell before gene introduction was used, and as a control of the antibody, an antibody different from the H-2K^{b}-specific antibody but matching in antibody class alone was used. As a result, L^{kb} cell showed certain expression of H-2K^{b} molecule (Fig. 4).

### Example 2

### Sensitization of precursor CTL and activation of antigen-specific CTL by the application of antigenic peptide to the tape-stripped skin.

In the following manner, it was confirmed that the activated epidermal Langerhans cell sensitizes precursor CTL and activates antigen-specific CTL by antigenic peptide pulse in the tape-stripped skin.
(i) One side of earlobe of a B6 mouse was disrupted by 10 repeats of stripping with the same adhesive tape as used in Example 1-(1), and antigenic peptides synthesized in Reference Example 1 were applied at 12, 24 and 48 hours later, wherein the antigenic peptides were respectively dissolved in 70% ethanol and applied in 10 µm per one side of the earlobe. At 1 week after the peptide application, cervical lymph node was removed, and lymphocytes were cultured for 2 days *in vitro* in an RPMI1640 medium containing a slight amount (5 U/ml) of IL-2. The L^{kb} cells labeled with ⁵¹Cr were pulsed with antigenic peptide (5 µmol) and L^{tk-} cells were used as a control. Cytotoxicity assay was performed using cultured lymphocytes as effector cells, and ⁵¹Cr-labeled and antigenic peptide-pulsed L^{kb} cells and L^{tk-} cells as target cells.
(ii) Variation in ratio of CTL in cervical lymph node
Table 1 shows variation in the total number of lymphocytes and the number of CD8-posititve cells in cervical lymph node upon determination at every antigenic peptide application. The antigenic peptides used were the three kinds recited in Reference Example 1. The antigenic peptide-pulsed epidermal Langerhans cell activated by tape stripping presented antigen in the lymph node, and sensitized and amplified the cells of immune system. In particular, the number of lymphocytes having a CD8 molecule, which is a CTL marker, showed an increase. The increase in the number of CD8-positive lymphocytes reached maximum at 12-24 hours after tape stripping, and matched well with the changes in the Langerhans cell counts in epidermis. Thus, it was strongly suggested that the activated epidermal Langerhans cells moved to the lymph node and activated CTL in the lymph node.

**Table 1**

| Peptide | Time after stripping | Total number of cells (x10⁷) | Number of (x10⁷) | CD8-positive cells (%) |
|---|---|---|---|---|
| HSV gp B | untreated | 0.82 | 0.25 | (20.5) |
| HSV gp B | 12 | 2.93 | 1.45 | (49.5) |
| HSV gp B | 24 | 1.86 | 0.86 | (46.2) |
| HSV gp B | 48 | 0.96 | 0.31 | (32.3) |
| VSV NP | untreated | 0.71 | 0.15 | (21.1) |
| VSV NP | 12 | 1.61 | 0.63 | (39.1) |
| VSV NP | 24 | 1.38 | 0.52 | (37.7) |
| VSV NP | 48 | 1.00 | 0.24 | (24.0) |
| Ovalbumin | untreated | 0.65 | 0.13 | (20.0) |
| Ovalbumin | 12 | 1.57 | 0.53 | (33.8) |
| Ovalbumin | 24 | 1.28 | 0.42 | (32.8) |
| Ovalbumin | 48 | 1.21 | 0.26 | (21.5) |
| (70% ethanol) | untreated | 0.73 | 0.19 | (26.0) |
| - | 12 | 0.72 | 0.19 | (26.4) |
| - | 24 | 0.77 | 0.20 | (26.0) |
| - | 48 | 0.80 | 0.23 | (28.8) |

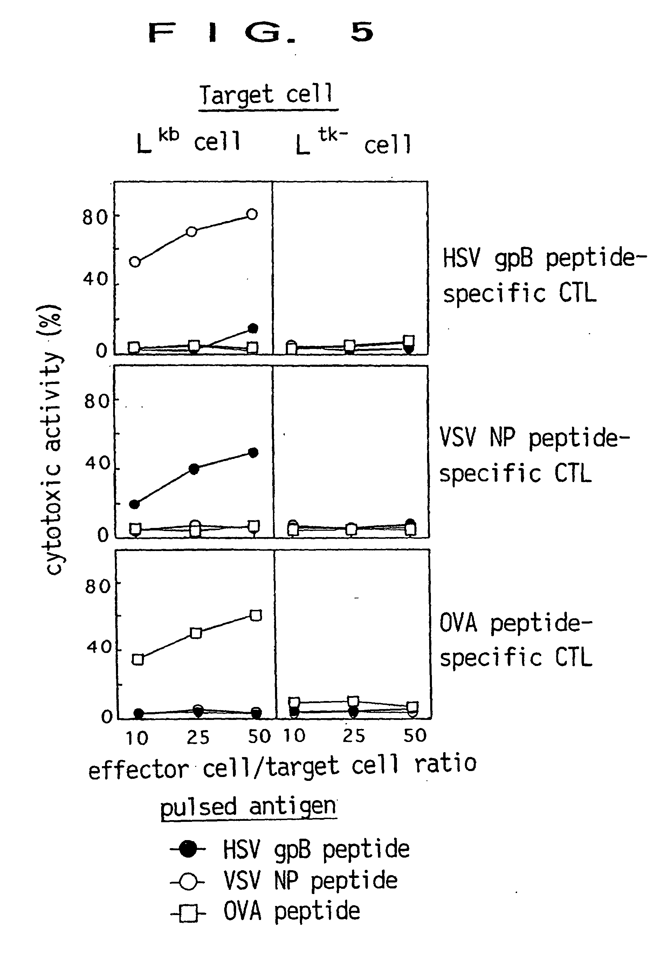

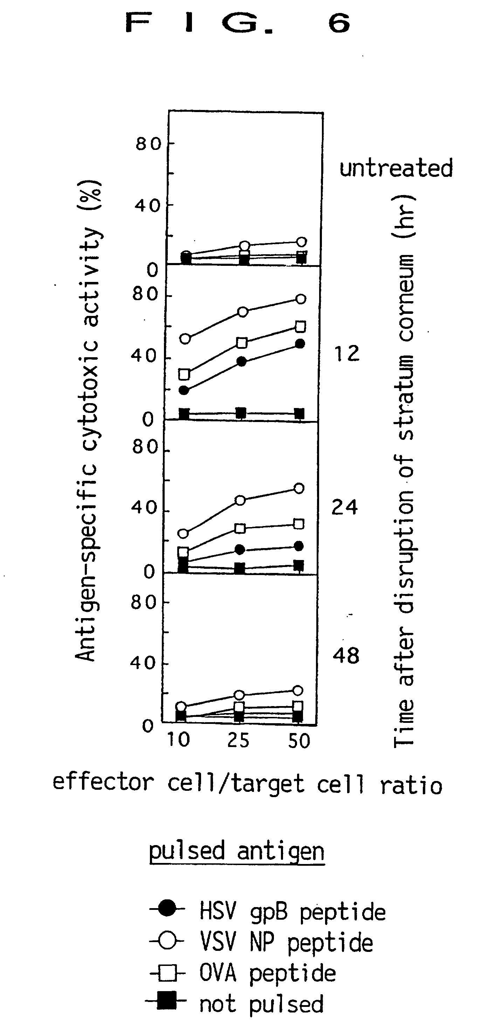

(iii) Activation of antigen-specific CTL
Cytotoxicity assay was performed using the effector cells derived from cervical lymphocytes obtained from the group that underwent separate application of antigenic peptides of HSV gp B, VSV NP and OVA at 12 hours after the tape stripping, the results of which are shown in Fig. 5. For example, CTL of cervical lymph node obtained from the group that underwent application of antigenic HSV gp B peptide showed amplification specific to HSV gp B, and recognized and certainly eradicated the cells presenting an H-2K^{b} - HSV gp B peptide complex alone. In the groups to which VSV NP peptide and OVA peptide were applied, cervical lymph node-derived CTL selectively damaged the cells presenting respective inoculated antigenic peptides. Naturally, greater effector cell/target cell ratios led to more ensured eradication of the target cells. (iii) Effect on sensitization of CTL by time-course application of antigenic peptide to the tape-shaped skin.
Cytotoxicity assay was performed using the effector cells derived from cervical lymphocytes obtained from the group that underwent separate application of antigenic peptides at 12, 24 and 48 hours after the tape stripping, the results of which are shown in Fig. 6. In every group, the peptide-pulsed epidermal Langerhans cells activated by tape stripping sensitized CTL in an antigen-specific manner in the lymph node and selectively damaged only the cells presenting respective antigenic peptides. It was clarified that, when antigenic peptide was applied at 12-24 hours after the tape stripping, the antigen-specific CTL were most strongly activated and amplified in the lymph node.

### Example 3

### Production of pressure sensitive adhesive tape containing antigenic peptide.

In a closed type reactor equipped with a stirrer were charged 2-methoxyethyl acrylate (70 parts by weight), N-vinyl-2-pyrrolidone (30 parts by weight), azoisobutyronitrile (0.17 part by weight) as a polymerization initiator, and a mixed solvent of distilled water:methanol:isopropanol (250 parts by weight, weight ratio 16:23:1). After displacement with nitrogen, the mixture was stirred for 1.5 hours while maintaining the temperature in the reactor at 60-62°C. Then, stirring at 75°C for 2 hours completed the reaction. The mixture was cooled to room temperature to give a viscous acrylic copolymer solution. To this polymer solution were added polyoxypropylenegycerol ether (10 parts by weight, average molecular weight 400) and trehalose (10 parts by weight, trademark TREHAOSE, available from HAYASHIBARA GROUP) and completely dissolved. The solution containing this acrylic copolymer was applied to a 6 µm thick flexible and thin polyester film, and dried at 130°C for 10 minutes to give a pressure sensitive adhesive tape having a 50 µm thick adhesive layer. Separately, the antigenic peptide HSV gp B obtained in Reference Example 1 was dissolved in 70% ethanol and applied to the surface of the adhesive layer of the pressure sensitive adhesive tape in an amount of 50 p mol per 1cm². Then, ethanol was evaporated to give a pressure sensitive adhesive tape inoculated with HSV gp B peptide. A release paper was adhered to this pressure sensitive adhesive tape to protect the adhesive layer surface until use.

### Example 4

### Sensitization of CTL and amplification of antigen-specific CTL by the application of antigenic peptide-containing pressure sensitive adhesive tape to the taped-stripped skin with disrupted stratum comeum barrier.

In the same manner as in Example 2 except that pressure sensitive adhesive tape inoculated with HSV gp B antigenic peptide, which had been obtained in Example 3, was applied immediately after tape stripping, instead of antigenic peptide, it was confirmed that epidermal Langerhans cells activated in an antigenic peptide specific manner by tape-stripping moved to the lymph node and activated antigenic peptide-specific CTL. The results are shown in Table 2.

**Table 2**

| Peptide | Time after stripping | Cytotoxic activity specific to HSV peptide-pulsed L cells (%) |
|---|---|---|
| HSV gp B | untreated | 0.2 ± 0.8 |
| HSV gp B | 12 | 45.3 ± 1.5 |
| HSV gp B | 24 | 37.0 ± 1.1 |
| HSV gp B | 48 | 3.5 ± 0.6 |
| - | untreated | 0.3 ± 0.5 |
| - | 12 | 1.1 ± 1.3 |
| - | 24 | 0.6 ± 1.2 |
| - | 48 | 0.5 ± 0.2 |

The epidermal Langerhans cells should have been activated after the passage of lead time necessary for the activation of epidermal Langerhans cells after tape stripping. Thus, when the antigenic peptide-containing pressure sensitive adhesive tape is applied to the skin surface upon tape stripping, antigenic peptide is inoculated and antigenic peptide-pulsed epidermal Langerhans cells move to the lymph node. As a result, antigenic peptide-specific CTL are activated. When compared to the inoculation method of Example 2, the antigenic peptide-specific CTL were amplified and activated faster, and when measured by setting the stripping time as time 0, the total amount of antigenic peptide-specific CTL amplified in a predetermined time period from the application of the antigenic peptide-containing pressure sensitive adhesive tape was found to have increased.

## Claims

1. A protective antigen-specific CTL activator in the form of a plaster, which comprises the protective antigen and a pharmaceutically acceptable carrier, the activator being suitable for an external application to an epidermal Langerhans cell surface activated by mechanically or chemically disrupting the stratum corneum of the epidermis.

2. The activator of claim 1, wherein said plaster comprises a support and an adhesive layer comprising the protective antigen in a releasable manner, said adhesive layer being laminated on one side of the support.

3. The activator of claim 2, wherein the surface of the adhesive layer is divided into two areas and either area comprises the protective antigen.

4. The activator of claim 1, further comprising a support and adhesive layers laminated on both sides of the support, wherein the adhesive layer on one side comprises the protective antigen in a releasable manner.

5. The activator of claim 1, wherein the protective antigen is selected from a pathogenic microorganism, a peptide to be an infection-protective antigen thereof and a malignant cell-specific mutated peptide.

6. The activator of claim 5, which is an agent for the prophylaxis and treatment of cancer or a disease caused by microbial infection.

7. Use of a protective antigen for the production of a protective antigen-specific CTL activator which is suitable to be externally applied to the surface of epidermal Langerhans cells activated by mechanically or chemically disrupting a stratum corneum of the epidermis.

8. The use of claim 7, wherein the activator is in the form of a plaster.

9. The use of claim 8, wherein the plaster comprises a support and an adhesive layer comprising the protective antigen in a releasable manner, said adhesive layer being laminated on one side of the support.

10. The use of claim 9, wherein the surface of the pressure sensitive adhesive tape is devided into two areas and the protective antigen is contained in one of the areas.

11. The use of claim 8, further comprising the use of a support and adhesive layers laminated on both sides of the support, said adhesive layer on one side comprising the protective antigen in a releasable manner, wherein the activator is in the form of a pressure sensitive adhesive tape.

12. The use of claim 7, wherein the protective antigen is at least one member selected from a pathogenic microorganism, a peptide to be an infection-protective antigen thereof and a malignant cell-specific mutated peptide.

13. The use of claim 7, wherein the activator is suitable for the prophylaxis and treatment of cancer or a disease caused by microbial infection.

## Patentansprüche

1. Aktivator für Schutzantigen-spezifische CTL in Form eines Pflasters, das das Schutzantigen und einen pharmazeutisch annehmbaren Träger umfasst, wobei der Aktivator für die externe Applikation auf die Oberfläche einer epidermalen Langerhans-Zelle geeignet ist, die durch mechanische oder chemische Zerstörung des Stratum corneum der Epidermis aktiviert wird.

2. Aktivator gemäß Anspruch 1, wobei das Pflaster einen Träger und eine Kleberschicht, die das Schutzantigen in freisetzbarer Weise enthält, umfasst, wobei die Kleberschicht auf eine Seite des Trägers laminiert ist.

3. Aktivator gemäß Anspruch 2, wobei die Oberfläche der Kleberschicht in zwei Bereiche aufgeteilt ist und jeder der beiden Bereiche das Schutzantigen umfasst.

4. Aktivator gemäß Anspruch 1, der weiterhin einen Träger und Kleberschichten, die auf beide Seiten des Trägers laminiert sind, umfasst, wobei die Kleberschicht auf einer Seite das Schutzantigen in freisetzbarer Weise enthält.

5. Aktivator gemäß Anspruch 1, wobei das Schutzantigen aus einem pathogenen Mikroorganismus, einem Peptid, das zu einem vor Infektionen schützenden Antigen des Mikroorganismus wird, und einem für maligne Zellen spezifischen mutierten Peptid ausgewählt ist.

6. Aktivator gemäß Anspruch 5, bei dem es sich um ein Mittel für die Prophylaxe und Behandlung von Krebs oder einer durch Mikrobeninfektion verursachten Krankheit handelt.

7. Verwendung eines Schutzantigens für die Herstellung eines Aktivators für CTL, die für das Schutzantigen spezifisch sind, wobei der Aktivator für die externe Applikation auf die Oberfläche einer epidermalen Langerhans-Zelle geeignet ist, die durch mechanische oder chemische Zerstörung des Stratum corneum der Epidermis aktiviert wird.

8. Verwendung gemäß Anspruch 7, wobei der Aktivator in Form eines Pflasters vorliegt.

9. Verwendung gemäß Anspruch 8, wobei das Pflaster einen Träger und eine Kleberschicht, die das Schutzantigen in freisetzbarer Weise enthält, umfasst, wobei die Kleberschicht auf eine Seite des Trägers laminiert ist.

10. Verwendung gemäß Anspruch 9, wobei die Oberfläche des Haftkleberbandes in zwei Bereiche aufgeteilt ist und das Schutzantigen in einem der Bereiche enthalten ist.

11. Verwendung gemäß Anspruch 8, die weiterhin die Verwendung eines Trägers und von Kleberschichten, die auf beide Seiten des Trägers laminiert sind, umfasst, wobei die Kleberschicht auf einer Seite das Schutzantigen in freisetzbarer Weise enthält, wobei der Aktivator in Form eines Haftkleberbandes vorliegt.

12. Verwendung gemäß Anspruch 7, wobei das Schutzantigen wenigstens ein Vertreter ist, der aus einem pathogenen Mikroorganismus, einem Peptid, das zu einem vor Infektionen schützenden Antigen des Mikroorganismus wird, und einem für maligne Zellen spezifischen mutierten Peptid ausgewählt ist.

13. Verwendung gemäß Anspruch 7, wobei der Aktivator für die Prophylaxe und Behandlung von Krebs oder einer durch Mikrobeninfektion verursachten Krankheit geeignet ist.

## Revendications

1. Activateur des lymphocytes T cytotoxiques protecteurs antigène-spécifiques sous la forme d'un pansement qui comprend l'antigène protecteur et un support pharmaceutiquement acceptable, l'activateur étant adapté à une application externe à la surface de cellules de Langherans épidermiques activé par la rupture mécanique ou chimique de la couche cornée de l'épiderme.

2. Activateur selon la revendication 1 dans lequel ledit pansement comprend un support et une couche adhésive comprenant l'antigène protecteur d'une façon libérable, ladite couche adhésive étant laminée sur un côté du support.

3. Activateur selon la revendication 2 dans lequel la surface de la couche adhésive est divisée en deux zones et chacune des zones comprend l'antigène protecteur.

4. Activateur selon la revendication 1, comprenant en plus un support et des couches adhésives laminées des deux côtés du support, dans lequel la couche adhésive sur un côté comprend l'antigène protecteur d'une façon libérable.

5. Activateur selon la revendication 1 dans lequel l'antigène protecteur est sélectionné à partir d'un microorganisme pathogène, un peptide destiné à devenir un antigène protecteur contre une infection et un peptide muté spécifique à une cellule maligne.

6. Activateur selon la revendication 5 qui est un agent pour la prophylaxie et le traitement du cancer ou d'une maladie causée par une infection microbienne.

7. Utilisation d'un antigène protecteur pour la production d'un activateur des lymphocytes T cytotoxiques protecteurs antigène-spécifiques qui est' adapté pour être appliqué de façon externe à la surface de cellules de Langherans épidermiques activé par la rupture mécanique ou chimique de la couche cornée de l'épiderme.

8. Utilisation selon la revendication 7 dans laquelle l'activateur est sous la forme d'un pansement.

9. Utilisation selon la revendication 8 dans laquelle le pansement comprend un support et une couche adhésive comprenant l'antigène protecteur d'une façon libérable, ladite couche adhésive étant laminée sur un côté du support.

10. Utilisation selon la revendication 9 dans laquelle la surface de la bande adhésive sensible à la pression est divisée en deux zones et l'antigène protecteur est contenu dans l'une des zones.

11. Utilisation selon la revendication 8, comprenant en plus l'utilisation d'un support et de couches adhésives laminées sur les deux côtés du support, ladite couche adhésive d'un côté comprenant l'antigène protecteur d'une façon libérable, dans laquelle l'activateur est sous la forme d'une bande adhésive sensible à la pression.

12. Utilisation selon la revendication 7 dans laquelle l'antigène protecteur est au moins un élément sélectionné à partir d'un microorganisme pathogène, un peptide censé devenir un antigène protecteur contre l'infection de celui-ci et un peptide muté spécifique à une cellule maligne.

13. Utilisation selon la revendication 7 dans laquelle l'activateur est adapté pour la prophylaxie et le traitement du cancer ou d'une maladie causée par une infection microbienne.


### SEQUENCE LISTING

(1) GENERAL INFORMATION:
   (i) APPLICANT:
      (A) NAME: Nitto Denko Corporation
      (B) STREET: 1-2, Shimohozumi 1-chome
      (C) CITY: Ibaraki-shi
      (D) STATE: Osaka
      (E) COUNTRY: Japan
      (F) POSTAL CODE (ZIP): 567-8680
   (ii) TITLE OF INVENTION: Killer T Cell Activator and Use Thereof
   (iii) NUMBER OF SEQUENCE: 3
   (iv) COMPUTER READABLE FORM:
      (A) MEDIUM TYPE: Diskette - 3.5 inch, 1.44 Mb storage
      (B) COMPUTER: IBM-PC compatible
      (C) OPERATING SYSTEM: PC-DOS/MS-DOS
      (D) SOFTWARE:
   (v) CURRENT APPLICATION DATA:
      (A) APPLICATION NUMBER:
      (B) FILING DATE:
      (C) CLASSIFICATION:
(2) INFORMATION FOR SEQ ID NO: 1:
   (i) SEQUENCE CHARACTERISTICS:
      (A) LENGTH: 8 amino acids
      (B) TYPE: amino acid
      (D) TOPOLOGY: linear
   (ii) MOLECULE TYPE:
      (A) DESCRIPTION: peptide
   (xi) SEQUENCE DESCRIPTION: SEQ ID NO: 1:
   Ser Ser Ile Glu Phe Ala Arg Leu
   5
(2) INFORMATION FOR SEQ ID NO: 2:
   (i) SEQUENCE CHARACTERISTICS:
      (A) LENGTH: 8 amino acids
      (B) TYPE: amino acid
      (D) TOPOLOGY: linear
   (ii) MOLECULE TYPE:
      (A) DESCRIPTION: peptide
   (xi) SEQUENCE DESCRIPTION: SEQ ID NO: 2:
   Arg Gly Tyr Val Tyr Gln Gly Leu
   5
(2) INFORMATION FOR SEQ ID NO: 3:
   (i) SEQUENCE CHARACTERISTICS:
      (A) LENGTH: 8 amino acids
      (B) TYPE: amino acid
      (D) TOPOLOGY: linear
   (ii) MOLECULE TYPE:
      (A) DESCRIPTION: peptide
   (xi) SEQUENCE DESCRIPTION: SEQ ID NO: 3:
   Ser Ile Ile Asn Phe Glu Lys Leu
   5